# EUROPEAN PATENT SPECIFICATION

(11) **EP 1 758 904 B1**
(45) Date of publication and mention of the grant of the patent: **03.06.2009**
(21) Application number: 04745116.6
(22) Date of filing: 11.06.2004
(51) Int. Cl.: C07D 471/14, A61K 31/407, A61P 35/00

(54) **FLAVOPEREIRINE DERIVATIVES FOR CANCER THERAPY**
FLAVOPEREIRINDERIVATE ZUR KREBSTHERAPIE
DERIVES DE FLAVOPEREIRINE POUR LE TRAITEMENT DU CANCER

(43) Date of publication of application: 07.03.2007
(73) Proprietor: DABUR PHARMA LTD., Safdarjung Hospital, New Delhi 110 029 (IN)
(72) Inventor: Seshagiri, V. Indian Institute of Chemical Biology, West Bengal (IN); Mukherjee, Rama Dabur Research Foundation, Ghaziabad 201 010 Uttar Pradesh (IN); Burman, Anand C. Dabur Research Foundation, Ghaziabad 201 010 Uttar Pradesh (IN); Gurjar, Mukund K. National Chemical Laboratory, Maharashtra (IN); Yadav, J. S. Indian Inst. of Chemical Technology, Andhra Pradesh (IN); Jaggi, Manu Dabur Research Foundation, Ghaziabad 201 010 Uttar Pradesh (IN); Singh, Anu T. Dabur Research Foundation, Ghaziabad 201 010 Uttar Pradesh (IN); Srivastava, Sanjay K. Dabur Research Foundation, Ghaziabad 201 010 Uttar Pradesh (IN); Jaisankar, P. Indian Institute of Chemical Biology, West Bengal (IN); Pal, Bikash Indian Institute of Chemical Biology, West Bengal (IN); Banerjee,Asish K. Indian Inst. of Chemical Biology, West Bengal (IN); Wakharkar, Radhika D. National Chemical Laboratory, Maharashtra (IN)
(74) Representative: Thomson, James B.
(86) International application number: PCT/IN2004/000167
(87) International publication number: WO 2005/121143

(56) References cited:
- EP-A- 0 059 817
- BASSLEER R ET AL: "EFFETS DE LA DIHYDROFLAVOPEREIRINE ET DE LA SEMPERVIRINE (ALCALOIDES DERIVES DE LA BETA-CARBOLINE) SUR DES CELLULES CANCEREUSES EN CULTURE EFFECTS OF DIHYDROFLAVOPEREIRINE AND SEMPERVIRINE (BETA-CARBOLINIUM ALKALOIDS) ON CANCER CELLS IN CULTURE" ANNALES PHARMACEUTIQUES FRANCAISES, MASSON, PARIS, FR, vol. 43, no. 1, 1985, pages 83-88, XP009000238 ISSN: 0003-4509
- ZIEGLER; SWEENY: "A synthesis of fihydrocorynantheol and 3-epi-dihydrocorynantheol" TETRAHEDRON LETTERS, no. 14, 1969, pages 1097-1100, XP002316751
- DATABASE BEILSTEIN BEILSTEIN INSTITUTE FOR ORGANIC CHEMISTRY, FRANKFURT-MAIN, DE; XP002316752 & MANNA ET AL.: SYNTH. COMMUN., vol. 25, no. 19, 1995, pages 3027-3034,

## Description

### Field of Invention

The present invention relates to novel derivatives of flavopereirine and methods for manufacture thereof. In particular, the present invention relates to novel derivatives of flavopereirine for use in the inhibition or prevention of the growth or multiplication of cancer cells, and to therapeutic compositions containing such compounds. The invention more specifically relates to derivatives of flavopereirine for use in the inhibition and / or prevention of cancer of the ovary, breast, prostate, pancreas, oral cavity, lung, colon, and larynx.

### Background of the Invention

Most aspects of the cancer problem revolve round the behaviour of DNA. DNA modification by ligand binding starts with the concept of intercalation. The most generally accepted criteria for intercalation come from direct techniques that measure some of the fundamental feature. One of them is that the plane of the aromatic chromophore of the bound drug must be parallel to that of the base pairs. In order to have better specificity and selectivity of action we have synthesized a new set of compounds viz. indolo[2,3-*a*]quinolizinium compounds which are better active than the alkaloid flavopereirine which is known to inhibit cancer producing cells. Previous syntheses of related compounds are disclosed in EP-A-0 059 817, in Annales Pharmaceutiques Francaises vol. 43, no. 1, 1985, pages 83-88, in Tetrahedron Letters, no. 14, 1969, pages 1097-1100 and in Synth. Commun. vol. 25, no. 19, 1995, page 3027.

### Summary of the Invention

This invention relates to the synthesis of new indolo[2,3-*a*]quinolizinium derivatives for cancer therapy having a general structural formula 1 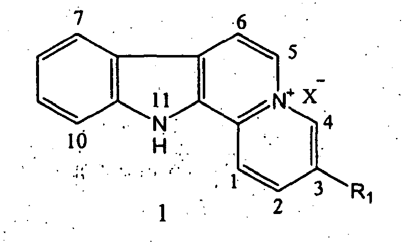 wherein R₁ represents alkyl (not ethyl), alkoxy, aryloxy, CH(OH)CH₃, CH(OAc)CH₃, CH(OCOC₆H₅)CH₃, CH(OBn)CH₃ and OBn, where Bn is CH₂C₆H₅ and X⁻ is a counter-anion; or having a general structural formula 8, 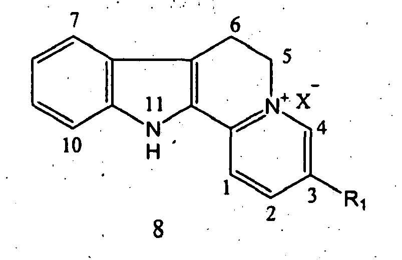 wherein R₁ represent alkyl (not ethyl), alkoxy, aryloxy, CH(OAc)CH₃, CH(OCOC₆H₅)CH₃ and CH(OBn)CH₃, where Bn is CH₂C₆H₅ and X⁻ is a counter-anion; or having one of the structures 22, 25, 26, 30 and 32

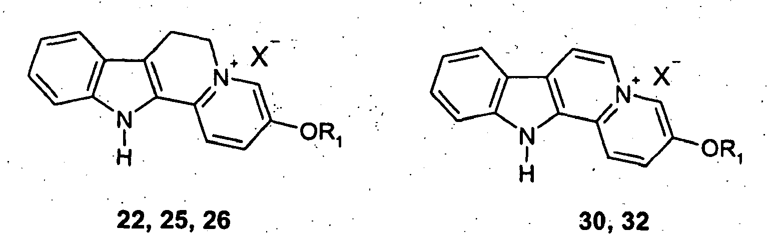

| **Compound** | **R₁** | **X** | **Compound** | **R₁** | **X** |
|---|---|---|---|---|---|
| **22** | H | HSO₄ | **30** | (CH₂)₃Br | ClO₄ |
| **25** | CH₂C₆H₄F[4-] | ClO₄ | **32** | CH₂C₆H₄F[4-] | ClO₄ |
| **26** | CH₂C₆H₄Br[4-] | Br | | | |

The present invention also relates to the synthesis of the new indolo[2,3-a]quinolizinium derivatives of formula 1 with anticancer activity.

The present invention also provides pharmaceutical compositions of the novel derivatives of flavopereirine based compounds or pharmaceutically acceptable salts of the flavopereirine based compounds useful for killing or inhibiting multiplication of cancer cells and for testing their bio-activity using cultured human cancer cells as the monitor.

### Detailed Description of the Invention

The present invention is directed to novel indolo[2,3-*a*]quinolizinium derivatives, methods for their manufacture and their use as new cancer agents.

### Preferred compounds of Formula 1 and 8 are shown below in Figure 1

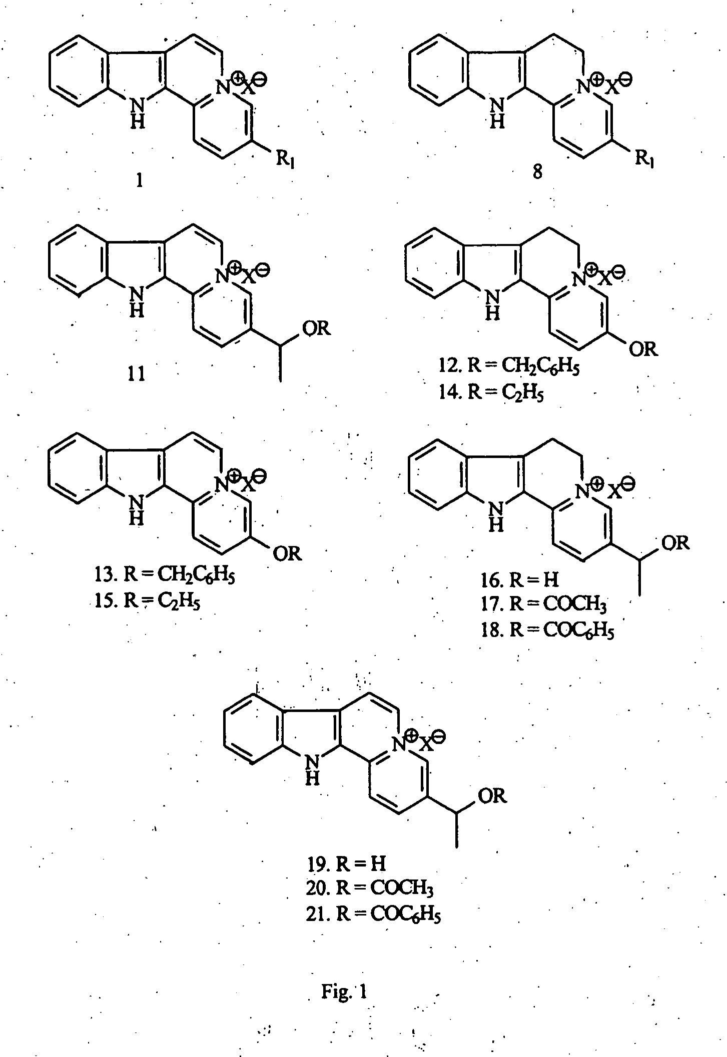

The present invention is also directed to the synthesis of compounds having the structural formula 1.

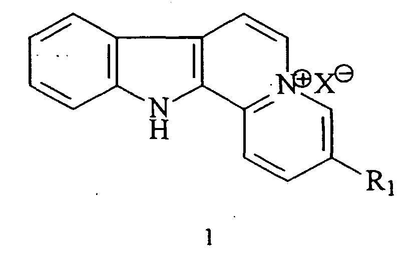

The preferred substitution pattern is aryloxy system in R₁ position.

In another preferred embodiment, the present invention contemplates employing compounds of formula 1 wherein R₁ is an aryloxy group.

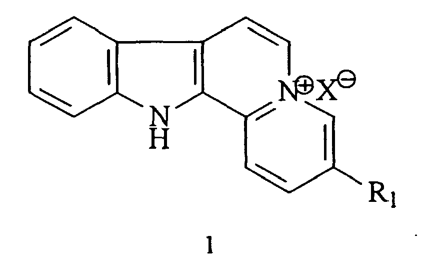

A typical synthesis of indolo[2,3-*a*]quinolizinium salts of the general formula 1, 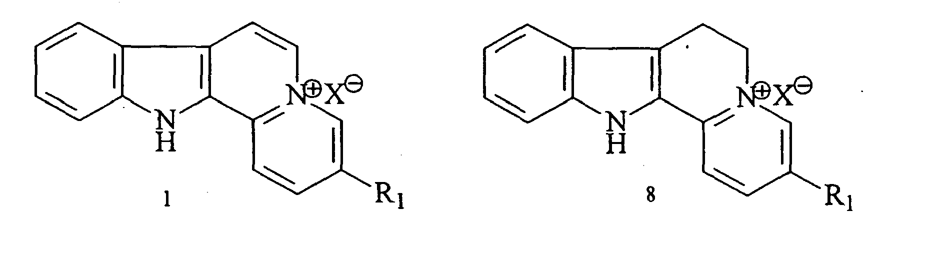 other derivatives of formula 8 and formula 11 are shown in figure 2 and 3, respectively

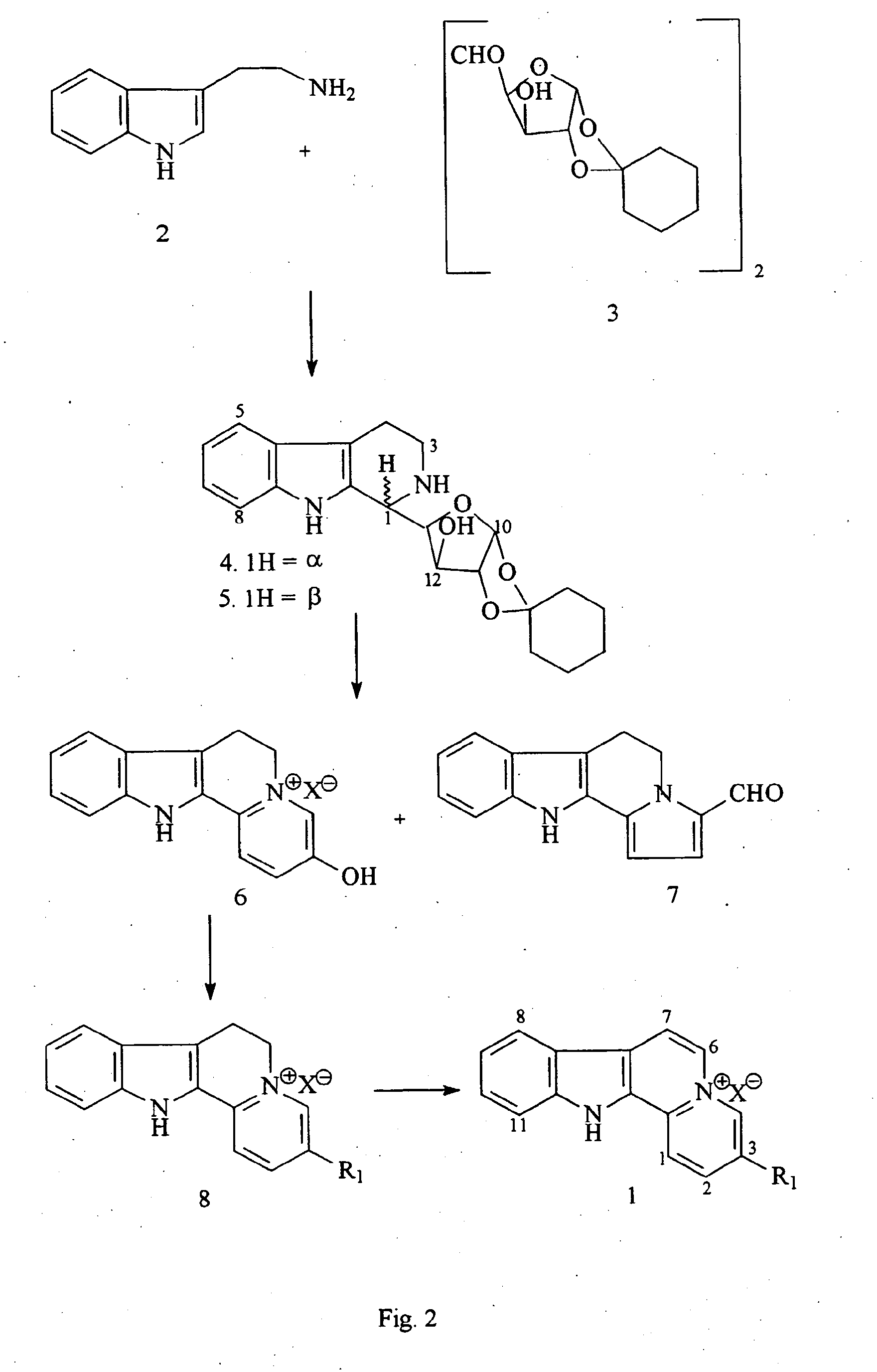

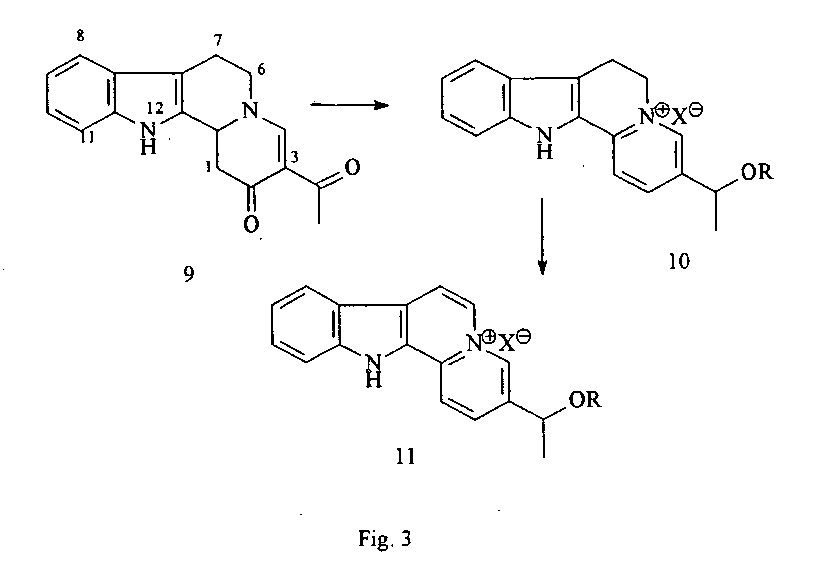

According to one embodiment of the invention, tryptamine of structure 2 is treated with di-(1,2-*O*-cyclohexylidene-α-D-xylo-pentadialdofuranose-5-hydrate)-5,5': 3',5-dianhydride of formula 3 in an inert solvent at a temperature in the range of 50°C - 150°C for a period in the range of 1 to 8 hrs., 1-(10,11-*O*-cyclohexylidene-12β-hydroxy-13β-tetrahydrofuranyl)tetrahydro-β-carboline of formula 4 and 1-(10,11-*O*-cyclohexylidene-12β-hydroxy-13α-tetrahydrofuranyl)tetrahydro-β-carboline of formula 5 are isolated and purified.

Thereafter, compounds of formula 4 and 5 are treated with acid at a temperature in the range of -10°C - 100°C for a period in the range 1 hr. to 8 hrs. and the compounds of formula 6 and formula 7 are recovered by conventional methods.

In an embodiment of the invention inert solvent may be benzene, toluene, xylene. In an embodiment of the invention acid may be H₂SO₄, HOAc, mixture of H₂SO₄ and HOAc.

Compounds of formula 6 were derivatised to compounds of formula 8 and converted to compounds of formula 1 by known methods. Alternately, they may be oxidized to compounds of formula 1 using different dehydrogenating agents preferably DDQ where DDQ is Dichloro dicyano benzoquinone.

Standard derivatisation techniques have been employed for converting compounds of formula 6.

In the case of compounds of general formula 1 where R₁ is CH(OH)CH₃ or CH(OAc)CH₃ or CH(OBn)CH₃ the derivatives may be prepared from the already known compound, 3-acetyl-1,2,6,7,12,12b-hexahydroindolo[2,3-*a*]quinolizine-2-one of formula 9.

The compound of formula 9 is reduced with NaBH₄ and the resultant mixture of alcohols is isolated by conventional methods. The mixture is refluxed with Hg(OAc)₂ in glacial HOAc to the compound of formula 10 which is derivatised before oxidation with DDQ to formula 11.

Representative salts of the compounds of formula 1 include but are not limited to the following: acetate, ascorbate, benzoate, citrate, oxalate, stearate, trifluoroacetate, succinate, tartarate, lactate, fumarate, gluconate, glutamate, phosphate/diphosphate, and valerate. The salts may be prepared in a conventional manner.

The present invention also provides a composition comprising a compound of the invention and a pharmaceutically acceptable carrier, diluent, or solvent. The composition may optionally and preferably contain pharmaceutically acceptable diluents, excipients, additives, fillers, lubricants, solvents, binders, stabilizers, and the like. Such diluents may include: RPMI 1649, buffered saline, isotonic NaCl, Ringer's solution, water, distilled water, polyethylene glycol (neat or in water), 2% Tween in water, dimethyl-sulfoxide to 50% in water, propylene glycol (neat or in water), phosphate buffered saline, balanced salt solution, glycerol, and other conventional fluids that are suitable, for intravenous administration: Pharmaceutical compositions, which provide from about 0.1 to 10 gram (preferably 0.5 to 5.0 gram) of the composition per unit dose are preferred and are conventionally prepared as tablets, lozenges, capsules, powders, aqueous or oily suspension, syrups, elixirs, and aqueous solutions. The nature of the pharmaceutical composition employed will, of course, depend on the desired route of administration.

The compositions may be used in a method of treatment for humans, mammals, or other animals suffering from cancer or other tumors. The method may suitably comprise, consist of, or consist essentially of administering a therapeutically effective dose of the pharmaceutical composition so as to kill or inhibit the multiplication of cancer or tumor cells. The invention relates more specifically to the use of the compounds of the invention in the manufacture of a medicament for use in the inhibition and/or prevention of cancer of the ovary, breast, prostate, pancreas, oral cavity, lung, colon, and larynx.

The methods noted above comprise, consist of, or consist essentially of administering systematically to the mammal a therapeutically effective combination of flavoperereine derivatives. An effective dose of flavoperereine derivatives or pharmaceutically acceptable salts of the flavoperereine derivatives ranges from 1mg / Kg. B. Wt to 300 mg / Kg. B. Wt (preferably 10-100 mg) / Kg. B. Wt) of the mammal, with the dose dependent on the effects sought, the manner of administration, and the cancer being treated. Systemic administration refers to oral, rectal, nasal, transdermal, and parental (i.e., intramuscular, intravenous and subcutaneous). In accordance with good clinical practice, it is preferred to administer the composition at a dose that will produce anticancer effects without causing undue harmful side effects. The composition may be administered either alone or as a mixture with other therapeutic agents such as 5-fluorouracil, methotrexate, etoposide, paclitaxel, taxotere, doxorubicin, daunarubicin, vincristine, vinblastine and other such known and established anticancer drugs.

An effective amount means that amount of a drug or pharmaceutical agent that will elicit the biological or medical response of a tissue, system, animal or human being sought.

Further chemical transformations to obtain the desired molecules were performed using standard methods and some of them have been presented in the following examples.

To further assist in the understanding of the present invention and not by way of limitation the following examples are presented to clearly describe the present invention.

### EXAMPLE 1

### Preparation of 1-(10,11-O-cyclohexylidene-12β-hydroxy-13β-tetrahydro-furanyl)tetrahydro-β-carboline of formula 4 and 1-(10,11-O-cyclohexylidene-12β-hydroxy-13α-tetrahydrofuranyl)tetrahydro-β-carboline of formula 5

A mixture of 1.6g (10mmol) of tryptamine of structure 2 and 2.5g (5.4mmol) of the sugar of structure 3 in a 1:1 aqueous methanolic solution (50ml) of 1N NaOAc and 1N HOAc was heated under stirring at 60°C. After most of the starting material has been consumed (TLC monitoring , 5hrs) the reaction mixture was evaporated under reduced pressure to remove the methanol. It was then neutralized with NaHCO₃ solution and extr-acted with chloroform. The extract was dried (Na₂SO₄), solvent evaporated and the residue chromotographed over basic alumina. Petroleum ether-chloroform (1:1) eluates were combined and concentrated to afford compound of formula 4 (mp 200-201°C; 2.04 g; yield, 55%). Chloroform-methanol (9.5:0.5) eluates were combined and concentrated to afford compound of formula 5 (mp 235-237°C; 1.11g; yield, 30%)

### Spectral data of compound 4:

**¹H NMR (CDCl₃):** δ 1.4 - 1.9 (brs, 10H), 2.64-2.84 (m, 2H, H-4), 2.9 - 3.20 (m, 2H, H-3), 3.3 - 3.6 (m, 2H, OH, NH), 4.24 (m, 1H, H-11), 4.4 - 4.60 (m, 3H, H-1, H-12, H-13), 6.04 (d, J = 5Hz, 1H, H-10), 7.04 - 7.60 (m, 4H, ArH), 8.44 (brs, 1H, NH).

**MS m/z (rel. int.):** 370 (M⁺, 90), 327 (25),271 (20), 255 (28), 237 (22), 213 (50), 199 (30), 184(84), 172(98), 171 (100).

### Spectral data of compound 5:

**¹H NMR (CDCl₃):** δ 1.3 - 1.9 (m, 10H), 2.6-2.9 (m, 2H, H-4), 3.01 - 3.40 (m, 2H, H-3), 4.36 - 4.39 (m, 2H, OH, NH), 4.45 - 4.55 (m, 2H), 5.98 (d, J = 3.3Hz, 1H, H-10), 7.04 - 7.20 (m, 3H, ArH), 7.47 (d, J=7.2 Hz, 1H), 8.20 (brs, NH, 1H).

**MS m/z (rel. int.):** 370 (M⁺, 95), 327 (20), 271 (20), 255 (30), 237 (22), 21.3 (50), 171 (100).

### EXAMPLE 2

### Preparation of 3-hydroxy-6,7-dihydroindolo[2,3-a]quinolizinium of formula 6 and 3-formyl - 5,6-dihydroindolo[2,3-a]quinolizinium of formula 7

A mixture (1 mmole, 370 mg) of compounds of formula 4 and 5 was stirred in a solution of 0.3 ml conc. H₂SO₄, 1ml water and 9ml acetonitrile at room temperature for 72hrs. The reaction mixture was monitored (TLC) and when no more starting materials remains it was diluted with water, evaporated to dryness and residue chromatographed over silica gel. CHCl₃- petroleum (9:1) eluates afforded the compound of formula 7 (mp 162-164°C; 24 mg; yield, 10%) Chloroform-methanol (4:1) eluates were combined and concentrated to afford compound of formula 6 isolated as its hydrogensulphate (mp 296-298°C; 267 mg; yield, 80%).

### Spectral data of compound 6:

**¹H NMR (DMSO-d₆):** δ 3.25 (t, J=7 Hz, 2H), 4.75 (t, J=7 Hz, 2H), 7.10 (t, J=7.5 Hz, 1H), 7.24 (t, J=7.5 Hz, 1H), 7.46 (d, J=8 Hz, 1H), 7.61 (d, J=8 Hz, 2H), 7.92 (d, J=9 Hz, 1H), 8.11 (s, 1H), 11.96 (brs, 1H, NH).

**MS m/z:** FAB 237 [M⁺]

### Spectral data of compound 7:

**¹H NMR (CDCl₃):** δ 3.4 (t, J=8 Hz, 2H, H-6), 4.8 (t, J=8 Hz, 2H, H-5), 6.38 (d, J=6 Hz, 1H, H-1), 7.02 (d, J=6 Hz, 1H, H-2), 7.08 - 7.68 (m, 4H, ArH), 8.34 (brs, 1H, NH), 9.58 (s, -CHO).

**MS m/z (rel. int):** 236 (M⁺, 100), 235(96), 207 (30), 206 (32).

### EXAMPLE 3

### Preparation of 3-benzyloxy-6,7-dihydroindolo[2,3-a]quinolizine of formula 12

To a solution of compound of formula 6 (334mg, 1mmol) in DMF (1ml) and triethyl amine (2ml), benzyl bromide (342mg, 2mmol) was added under stirring at room temperature. The reaction mixture was stirred for 8hrs. The solvents were removed under reduced pressure and the residue was chromatographed over silica gel. Chloroform-methanol (4:1) eluates afforded compound of formula 12 isolated as its perchlorate (mp 278-280°C; 338 mg; yield, 80%).

### Spectral data of compound 12:

**¹H NMR (DMSO-d₆):** δ 3.36 (t, J=7 Hz, 2H), 4.90 (t, J=7 Hz, 2H), 5.35 (s, 2H), 7.16 (t, J=7.5 Hz, 1H), 7.34 (t, J=7.5 Hz, 1H), 7.41 - 7.54 (m, 6H), 8.20 (d, J=9 Hz, 1H), 8.40 (d, J=9 Hz, 1H), 8.99 (s, 1H), 12.21 (brs, 1H, NH).

**MS m/z:** FAB 327 [M⁺]

### EXAMPLE 4

### Preparation of 3-benzyloxyindolo[2,3-a]quinolizinium of formula 13

To a solution of 100mg (0.23mmol) of the perchlorate salt of the compound of formula 12 in glacial HOAc (10ml) was added 150mg (0.6mmol) of DDQ at a time and the reaction mixture heated over steam bath for 6hrs. The reaction mixture on cooling afforded crystals of the perchlorate of compound of formula 13 (296-298°C; 68 mg; yield 70%).

### Spectral data of compound 13:

**¹H NMR (DMSO-d₆):** δ 5.39 (s, 2H), 7.36 - 7.55 (m, 5H), 7.58 (d, J=7.5 Hz, 1H), 7.72 (t, J=7.5 Hz, 1H), 7.85 (d, J=7.5 Hz, 1H), 8.3 (d, J=7.5 HZ, 1H); 8.43 (d, J= 7.5 Hz, 1H), 8.83 (d, J=7.5 Hz, 1H), 8.90 (d, J=9 Hz, 1H), 9.01 (d, J=9Hz, 1H), 9.38 (s, 1H), 13.36 (brs, 1H, NH).

**MS m/z:** FAB 325 [M⁺]

### EXAMPLE 5

### Preparation of 3-ethoxy-6,7-dihydroindolo[2,3-a]quinolizinium of formula 14

To a solution of compound of formula 6 (334mg, 1mmol) in DMF (1 ml) and triethyl amine (2ml), ethyl bromide (218mg, 2mmol) was added under stirring at, room temperature. The reaction mixture was stirred for 8hrs. The solvents were removed under reduced pressure and the residue was chromatographed over silica gel. Chloroform-methanol. (4:1) eluates afforded the compound of formula 14 isolated as its perchlorate (mp 305°C (dec.); 291 mg; yield, 80%).

### Spectral data of compound 14:

**¹H NMR (DMSO-d₆):** δ 1.42 (t, J=6.9 Hz, 3H), 4.28 (q, J=6.9 Hz, 2H), 3.34 (t, J=6.9 Hz, 2H), 4.88 (t, J=6.9 Hz, 2H), 7.16 (t, J=7.1 Hz, 1H), 7.33 (t, J=7.1 Hz, 1H), 7.52 (d, J=8.1 Hz, 1H), 7.70 (d, J=8.1 Hz, 1H), 8.16 (d, J=9 Hz, 1H), 8.29 (d, J=9 Hz, 1H), 8.84 (s, 1H), 12.17 (brs, NH, 1H).

**MS m/z:** FAB 265 [M⁺]

### EXAMPLE 6

### Preparation of 3-ethoxyindolo [2,3-a]quinolizinium of formula 15

To a solution of 100mg (0.28mmol) of the compound of formula 14 in glacial HOAc (10ml) was added 150mg (0.6mmol) of DDQ at a time and the reaction mixture heated over steam bath for 6hrs. The reaction mixture on cooling afforded crystals of the perchlorate of compound of formula 15 (310°C (dec.); 75 mg; yield, 75%).

### Spectral data of compound 15:

**¹H NMR (DMSO-d₆):** δ 1.48 (t, J=6.9 Hz, 3H), 4.32 (q, J=6.9 Hz, 2H), 7.46 (t, J=7.5 Hz, 1H), 7.71 (t, J=7.5 Hz, 1H), 7.83 (d, J=8.1 Hz, 1H), 8.23 (dd, J=1.5 Hz, 9.0 Hz, 1H), 8.40 (d, J=7.8 Hz, 1H), 8.78 (d, J=6.9 Hz, 1H), 8.82 (d, J=9.0 Hz, 1H), 8.95 (d, J=6.9 Hz, 1H), 9.19 (d, J=1.5 Hz, 1H), 13 .31 (brs, NH, 1H).

**MS m/z:** FAB 263 [M⁺]

### EXAMPLE 7

### Preparation of 3-(α-hydroxyethyl)-6,7-dihydroindolo[2,3-a]quinolizinium of formula 16

The known compound of formula 9 (2.80gm, 10mmol) obtained from 1-methyl-3,4-dihydro-β-carboline and hydroxymethylene acetylacetone was taken in methanol (40ml) and NaBH₄ (1.90gm, 50mmol) was added in portions at room temperature. After all the starting material had been reduced (TLC monitoring), water was added to the reaction mixture and it was extracted with chloroform. The extract was dried (Na₂SO₄), evaporated to dryness in vacuum to a solid residue. This residue was dissolved in HOAc (50ml) and Hg(OAc)₂ (6.37gm, 20ml) was added to it. The mixture was refluxed for 5hrs. , Cooled and the solvent removed by rotavapor. The residue was diluted with water and H₂S was passed into it till there was no further separation of HgS. The solution was filtered over a bed of celite and the filtrate evaporated to dryness. The residue was chromatographed over silica gel. Chloroform-MeOH (95:5) eluates afforded the compound of formula 16 isolated as perchlorate salt (mp 300-302°C; 2.91g; yield, 80%).

### Spectral data of compound 16:

**¹H NMR (DMSOd₆):** δ 1.46 (d, J=7 Hz, 3H), 3.37 (t, J=7 Hz, 2H), 4.94 (t, J=7 Hz, 2H), 4.98 (q, J=7 Hz, 1H), 6.00 (d, J=4 Hz, 1H, OH), 7.16 (t, J=7 Hz, 1H), 7.3 (t, J=7 Hz, 1H), 7.54 (d, J=8 Hz, 1H), 7.71 (d, J=8 Hz, 1H), 8.46 (d, J=8 Hz, 1H), 8,52 (d, J=8 Hz, 1H), 8.95 (s, 1H), 12.92 (brs, 1H, NH).

**MS m/z (rel. int.):** 265 (M⁺, 9), 264(47), 263 (100), 247 (19), 219 (12), 218 (7).

### EXAMPLE 8

### Preparation of 3-(α-acetoxyethyl)-6,7-dihydroindolo [2,3-a]quinolizinium of formula 17

A solution of compound 16 (365mg, 1mmol) in pyridine (1ml) and acetic anhy-dride (0.5ml) was heated for 3hrs over steam bath. The reaction mixture was then concentrated to remove both excess pyridine and acetic anhydride in a rotary evaporator.The residue was chromatographed over silicagel. Chloroform-MeOH (99:1) eluates were combined, concentrated to afford the compound of formula 17 isolated as its perchlorate (mp 298-300°C (dec.); 386 mg; yield, 95%).

### Spectral data of compound 17:

**¹H NMR (DMSO-d₆):** δ 1.60 (d, J=7 Hz, 3H), 2.14 (s, 3H), 3.40 (t, J=7 Hz, 2H), 4.96 (t, J=7 Hz, 2H), 5.98 (q, J=7 Hz, 1H), 7.08 - 7.84 (m, 4H), 8.46 - 8.74 (m, 2H), 9.12 (s, 1H), 12.96 (brs, 1H, NH).

**MS m/z:** FAB 307 [M⁺]

### EXAMPLE 9

### Preparation of 3-(α-benzoyloxyethyl)-6,7-dihydroindolo[2,3-a]quinolizinium of formula 18

A solution of compound 16 (365mg, 1mmol) in pyridine (1ml) and benzoyl chloride (0.5ml) was heated for 3hrs over steam bath. The reaction mixture was then concentrated to remove both excess pyridine and benzoyl chloride in a rotary evaporator.The residue was chromatographed over silica gel. Chloroform-MeOH (99:1) eluates were combined to afford the compound of formula 18 isolated as chloride salt (mp 288-289°C; 364 mg; yield, 90%).

### Spectral data of compound 18:

**¹H NMR (DMSO-d₆):** δ 1.72 (d, J=6.5 Hz, 3H), 3.30 (t, J=7.5 Hz, 2H), 4.93 (t, J=7.5 Hz, 2H), 6.21 (q, J=6.5 Hz, 1H), 7.17 (t, J=7.5 Hz, 1H), 7.38 (t, J=7.5 Hz, 1H), 7.5 - 7.75 (m, 5H), 8.05-8.09 (m, 2H), 8.23 (d, J=7.5 Hz, 1H), 8.73 (d, J=8 Hz, 1H), 9.15 (s, 1H), 12.33 (brs, 1H).

**MS m/z:** FAB 369 [M⁺]

### EXAMPLE 10

### Preparation of 3-(α-hydroxyethyl)indolo[2,3-a]quinolizinium of formula 19

To a solution of 100mg (0.28mmol) of the compound of formula 16 in glacial HOAc (10ml) was added 150mg (0.6mmol) of DDQ at a time and the reaction mixture heated over steam bath for 6hrs. Addition of few drops of HClO₄ to the reaction mixture afforded compound of formula 19 as perchlorate salt (mp 288-290°C; 83 mg; yield, 82%)

### Spectral data of compound 19:

**¹H NMR (DMSO-d₆):** δ 1.53 (d, J=6.5 Hz, 3H), 5.05-5.10 (m, 1H), 5.96 (d, J=4.5 Hz, 1H), 7.49 (t, J=7 Hz, 1H), 7.75 (t, J=7 Hz, 1H), 7.88 (d, J=8.4 Hz, 1H), 8.46 (d, J=7.8 Hz, 1H), 8.84 (d, J=7 Hz, 1H), 8.9 (d, J=9 Hz, 1H), 9.15 (d, J=7 Hz, 1H), 9.4 (brs, 1H).

**MS m/z:** FAB 263 [M⁺]

### EXAMPLE 11

### Preparation of 3-(α-acetoxyethyl)indolo[2,3-a]quinolizinium of formula 20

To solution of 100mg (0.24mmol) of the compound of formula 17 in glacial HOAc (10ml) was added 150mg (0.6mmol) of DDQ at a time and the reaction mixture heated over steam bath for 6hrs. Addition of few drops of HClO₄ to the reaction mixture afforded compound of formula 20 as perchlorate salt (mp 270-272°C; 68 mg; yield, 70%).

### Spectral data of compound 20:

**¹H NMR (DMSO-d₆):** δ 1.66 (d, J=6.5 Hz, 3H), 2.16 (s, 3H), 6.1 (q, J=6.5 Hz, 1H), 7.5 (t, J=7.5 Hz, 1H), 7.76 (t, J=7.5 Hz, 1H), 7.88 (d, J=8 Hz, 1H), 8.46 (d, J=7.8 Hz, 1H), 8.53 (d, J=9 Hz, 1H), 8.88 (d, J=7 Hz, 1H), 8.95 (d, J=9 Hz, 1H), 9.12 (d, J=7 Hz, 1H), 9.5 (brs, 1H).

**MS m/z:** FAB 305 [M⁺]

### EXAMPLE 12

### Preparation of 3-(α-benzoyloxyethyl)indolo[2,3-a]quinolizinium of formula 21

To solution of 100mg (0.21mmol) of the compound of formula 18 in glacial HOAc (10ml) was added 150mg (0.6mmol) of DDQ at a time and the reaction mixture was heated over steam bath for 6hrs. Addition of few drops of HClO₄ to the reaction mixture afforded compound of formula 21 as perchlorate salt (mp 298-300°C; 72 mg; yield, 74%).

### Spectral data of compound 21:

**¹H NMR (DMSO-d₆):** δ 1.81 (d, J=6.5 Hz, 3H), 6.37 (q, J=6.5 Hz, 1H), 7.48 - 7.78 (m, 5H), 7.88 (d, J=8.1 Hz, 1H), 8.11 (d, J=7.2 Hz, 2H), 8.47 (d, J=7.8 Hz, 1H), 8.65 (d,. J=8.4 Hz, 1H), 8.88 (d, J=6.9 Hz, 1H), 9.05 (d, J=8.4 Hz, 1H), 9.17 (d, J=6.9 Hz, 1H), 9.63 (s, 1H), 13.6 (brs, 1H, NH).

**MS m/z:** FAB 367 [M⁺]

### EXAMPLE 13

### In Vitro Cytotoxicity of the Flavopereirine derivatives

A number of the Flavopereirine derivatives were tested for cytotoxicity against nine (9) human tumor cell lines. Briefly, a three day MTT cytotoxicity assay was performed, which is based on the principle of uptake of MTT (3-(4,5-dimethylthiazol-2-yl)-2,5-diphenyl tetrazolium bromide), a tetrazolium salt, by the metabolically active cells where it is metabolized by active mitochondria into a blue colored formazan product that is read spectrophotometrically. MTT was dissolved in phosphate buffered saline with a pH of 7.4 to obtain an MTT concentration of 5mg/ml; the resulting mixture was filtered through a 0.22-micron filter to sterilize and remove a small amount of insoluble residue. For each type of tumor cell, 10000 to 15000 cells were seeded in a 96-well culture plate and incubated with the individual Flavopereirine derivatives in concentration ranging from 1 -20ug/ml in a CO₂ incubator for a total of 72 hours. Control cells not treated with the Flavopereirine derivatives were similarly incubated. The assay was terminated by adding 100 ug (20 ul) of MTT to each well, then incubating for additional one hour, and finally adding 50 ul of 10% SDS-0.01N HCl to each well to lyse the cells and dissolve formazan. After incubating for one hour, the plate was read spectrophotometrically at 540 nm and the percentage of cytotoxicity calculated using the following formula:

Cytotoxicity percentage = 100 x [1-(X/R₁)], where X = (absorbance of treated sample at 540 nm) - (absorbance of blank at 540 nm) R₁ = absorbance of control sample at 540 nm.

The IC₅₀ Values of the cytotoxicity defined as the concentration at which 50 % of the cells are killed in vitro was calculated for each cell line treated with each of the Flavopereirine derivatives:

The cell lines are ovary (PA-1), breast (MCF-7), prostate (DU145), pancreas (MiaPaCa.2), oral cavity (KB), lung (L132), colon (SW620), and larynx (HeP2). The flavopereirine derivatives tested for their cytotoxicity are shown in table 1. The ED50 values of in vitro cytotoxicity of the flavoperereine derivatives of this invention are shown in the Table 2.

**Table 1: Flavopereirine derivatives (22-33)**

| | | | | | |
|---|---|---|---|---|---|
| 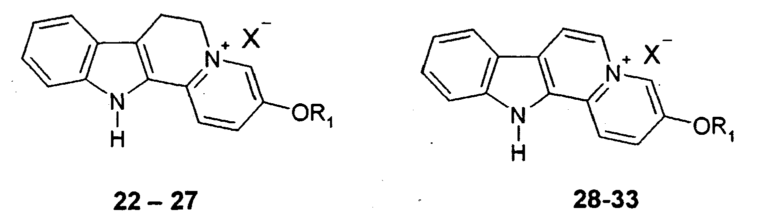 | | | | | |

| **Compound** | **R₁** | **X** | **Compound** | **R₁** | **X** |
|---|---|---|---|---|---|
| **22** | H | HSO₄ | **28** | CH₂C₆H₅ | ClO₄ |
| **23** | CH₂C₆H₅ | ClO₄ | **29** | CH₂CH₃ | ClO₄ |
| **24** | CH₂CH₃ | ClO₄ | **30** | (CH₂)₃Br | ClO₄ |
| **25** | CH₂CeH₄F[4-] | ClO₄ | **31** | CH₂C₆H₅ | Br |
| **26** | CH₂C₆H₄Br[4-] | Br | **32** | CH₂C₆H₄F[4-] | ClO₄ |
| **27** | (CH₂)₃CH₃ | Br | **33** | (CH₂)₃CH₃ | Br |

**Table 2: IC₅₀ (µg/mL) of Flavoperereine derivatives in human tumor cell lines**

| **Compound** | **Cell line** | | | | | | | | |
|---|---|---|---|---|---|---|---|---|---|
| | **PA1 Ovarian** | **MCF7 Breast** | **DU145 Prostate** | **MiaPaca2 Pancreas** | **KB Oral** | **L132 Lung** | **Hep2 Larynx** | **SW620 Colon** | **ECV-304 Endothelial** |
| **22** | >100 | >100 | >100 | >100 | >100 | >100 | >100 | >100 | ND |
| **23** | 31 | 5 | 10 | 10 | 12 | 20 | 17 | 11 | 16 |
| **24** | 38 | ND | ND | ND | 50 | 20 | 89 | 30 | 64 |
| **25** | 36 | 34 | 41 | 30 | 8 | 9 | 44 | 44 | 24 |
| **26** | >100 | >100 | 7.5 | 9.5 | 6 | ND | 24 | >100 | 12 |
| **27** | 4.7 | 6 | 9 | 7.5 | 7 | ND | 5 | 4 | ND |
| **28** | 1.5 | 5 | 2 | 2 | 2 | 2 | 1.5 | <1 | 4 |
| **29** | 5 | ND | ND | ND | 10 | 15 | 8.0 | 10 | 4 |
| **30** | 2 | 6 | 7.5 | 4 | 10 | 10 | 1.5 | 6 | 6 |
| **31** | 4 | 4 | 4.5 | <1 | <1 | 4.5 | 3 | 5 | 13.5 |
| **32** | 6 | <1 | 2 | <1 | <1 | <1 | 4 | 5 | 24 |
| **33** | <1 | 3 | 5 | 2 | 1.6 | ND | 2.4 | 1.4 | ND |

A Pub Med search of the National Library of Medicine was carried out to determine the relevance of cell lines used by us for determining the anticancer activity of the peptides. While DU145 (human prostate) showed 542 "hits" when searched with reference to cancer, other human cancer cell lines used by us also showed large number of hits (1063 for MCF-7 and 182 for PA-1). This clearly shows the extensive use of these cell lines in cancer research. Further, it is a common and standard practice and norm for testing molecules for anticancer activity in vitro on human tumor cell lines. (Br J Cancer. 2001 May 18; 84(10): 1289-90 (Flasks, Fibres & Flanks - Preclinical tumor models for predicting clinical antitumor activity). The authors report that in vitro activity against 6 or more lung or breast cancer cell lines does predict xenograft activity against these tumor types. In articles "Semin Oncol 1992 Dec.; 19(6): 622-38 (The National Cancer Institute: cancer drug discovery and development program) and "Jpn J Antibiot 1977 Dec.;30 Suppl:35-40 (Antitumor screening procedures of the National Cancer Institute)" extensive use of human tumor cell lines for identification of potential cytotoxic drugs is described.

## Claims

1. A compound selected from (i) compounds of formula 1 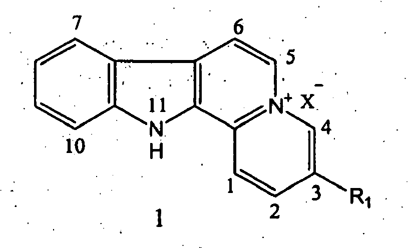 wherein R₁ represents alkyl (not ethyl), alkoxy, aryloxy, CH(OH)CH₃, CH(OAc)CH₃, CH(OCOC₆H₅)CH₃, CH(OBn)CH₃ and OBn, where Bn is CH₂C₆H₅ and X⁻ is a counter-anion;
(ii) compounds of formula 8, wherein R₁ represent alkyl (not ethyl), alkoxy, aryloxy, CH(OAc)CH₃, CH(OCOC₆H₅)CH₃ and CH(OBn)CH₃, where Bn is CH₂C₆H₅ and X⁻ is a counter-anion; 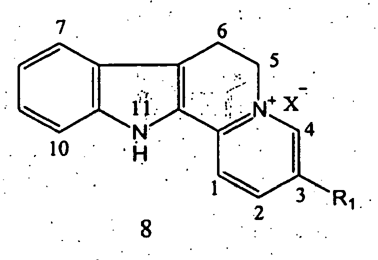
(iii) compounds 22, 25, 26, 30 and 32 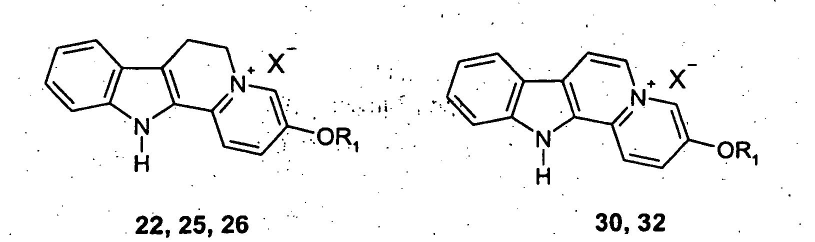
| **Compound** | **R₁** | **X** | **Compound** | **R₁** | **X** |
|---|---|---|---|---|---|
| **22** | H | HSO₄ | **30** | (CH₂)₃Br | ClO₄ |
| **25** | CH₂C₆H₄F[4-] | ClO₄ | **32** | CH₂C₆H₄F[4-] | ClO₄ |
| **26** | CH₂C₆H₄Br[4-] | Br | | | |

2. A compound according to claim 1, having formula 13 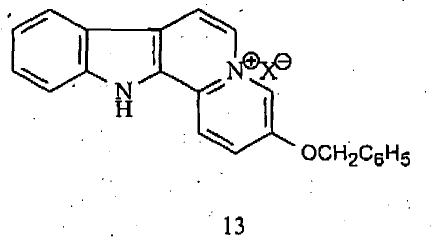 and is 3-benzyloxyindolo[2,3-*a*]quinolizinium salt.

3. A compound according to claim 1, having formula 15 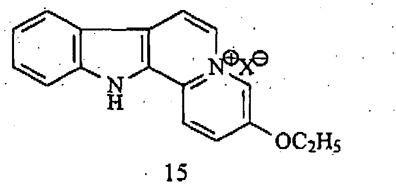 and is 3-ethoxyindolo[2,3-*a*]quinolizinium salt.

4. A compound according to claim 1, having formula 19 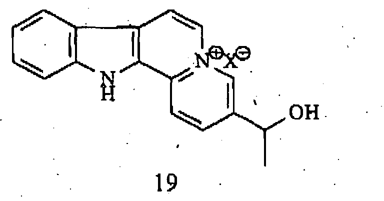 and is 3-(α-hydroxyethyl)indolo[2,3-*a*]quinolizinium salt.

5. A compound according to claim 1, having formula 20 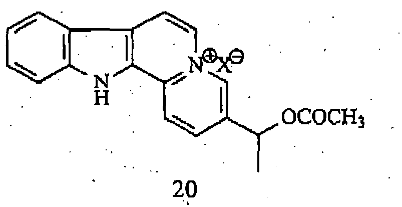 and is 3-(α-acetoxyethyl)indolo[2,3-*a*]quinolizimum salt.

6. A compound according to claim 1, having formula 21 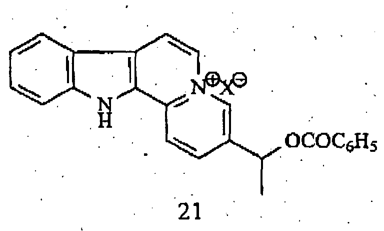 and is 3-(α-benzoyloxyethyl)indolo[2,3-*a*]quinolizinium salt.

7. A compound according to claim 1, having formula 14 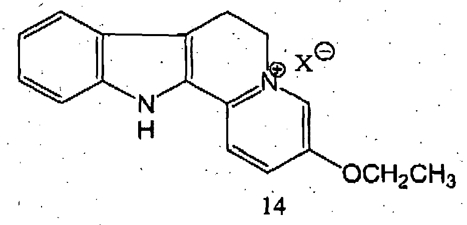 and is 3-ethoxy-5,6-dihydroindolo[2,3-*a*]quinolizinium salt.

8. A compound according to claim 1, having formula 17 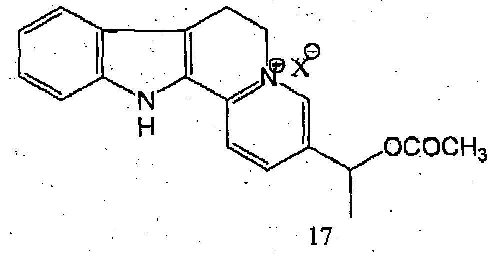 and is 3-(α-acetoxyethyl)-5,6-dihydroindolo[2,3-*a*]quinolizinium salt.

9. A compound according to claim 1, having formula 18 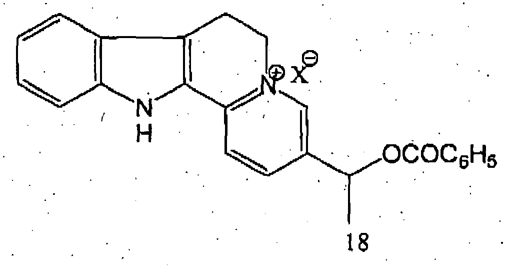 and is 3-(α-benzoyloxyethyl)-5,6-dihydroindolo[2,3-*a*]quinolizinium salt.

10. A composition comprising a compound of any one of claims 1-9, and a pharmaceutically acceptable additive, diluent, excipient, solvent, binder, stabilizer, carrier, filler or lubricant.

11. A composition as claimed in claims 1, 2-6 and 10, which provides 0.1 to 10 gram,per unit dose of the compound of formula 1.

12. A composition as claimed in claims 1, 7-9 and 10, which provides 0.1 to 10 gram per unit dose of the compound of formula 8.

13. The composition as claimed in claims 10-12 in the form of a tablet, lozenge, capsule, powder, aqueous or oily suspension, syrup, elixir, implant or aqueous solution.

14. A compound according to any one of claims 1-9 for use in a method of treating a patient with cancer of the ovary, breast, prostate, pancreas, oral cavity, lung, colon, or larynx, said method comprising administering an effective amount of a compound as claimed in any one of the claims 1-9 or a composition as claimed in any one of claims 10-13 to the patient in need thereof.

15. Use of a compound according to any one of claims 1-9 in the manufacture of a medicament for use in a method for treating a patient with cancer of the ovary, breast, prostate, pancreas, oral cavity, lung, colon, or larynx, said method comprising administering an effective amount of a compound as claimed in any one of the claims 1-9 or a composition as claimed in any one of claims 10-13 to the patient in need thereof

16. Use as claimed in claim 15, wherein said patient is a human, mammal or other animal.

17. Use as claimed in claims 15 and 16 wherein the dosage for humane is in the range of 1mg/Kg. B. Wt to 3 00 mg*l*Kg. B. Wt.

18. Use as claimed in claims 15-17, wherein the compound is administered to the patient systemically.

19. Method for the synthesis of indolo[2,3-*a*]quinolizinium salts of the general formula 1 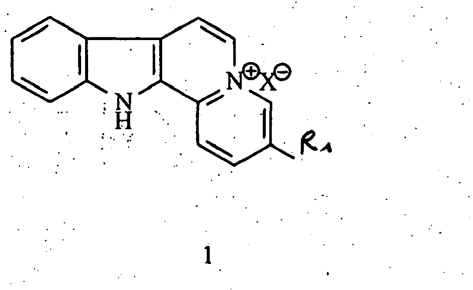 wherein R₁ represents alkyl (not ethyl), alkoxy, aryloxy, CH(OH)CH₃, CH(OAc)CH₃, . CH(OBn)CH₃, where Bn is CH₂C₆H₅ and X⁻ is a counter-anion, which comprises
treating, a tryptamine of formula 2 with di-(1,2-*O*-cyclohexylidene-α-D-xylopentadialdofuranose-5-hydrate)-5,5', 3',5'-dianhydride of formula 3 in an inert solvent at a temperature in the range of 50°C - 150°C for a period in the range of 1 to 8 hrs.,
isolating and purifying 1-(10,11-*O*-cyclohexylidene-12β-hydroxy-13β-tetrahydrofuranyl) tetrahydro-β-carboline of formula 4 and 1-(10,11-O-cyclohexylidene-120-hydroxy-13α-tetrahydrofuranyl)tetrahydro-β-carboline of formula 5 so produced,
treating said compounds to form compounds of formula 6 and formula 7,
derivatising said compounds of formula 6 to compounds of formula 8
and converting said compounds of formula 8 to compounds formula 1 by known methods:

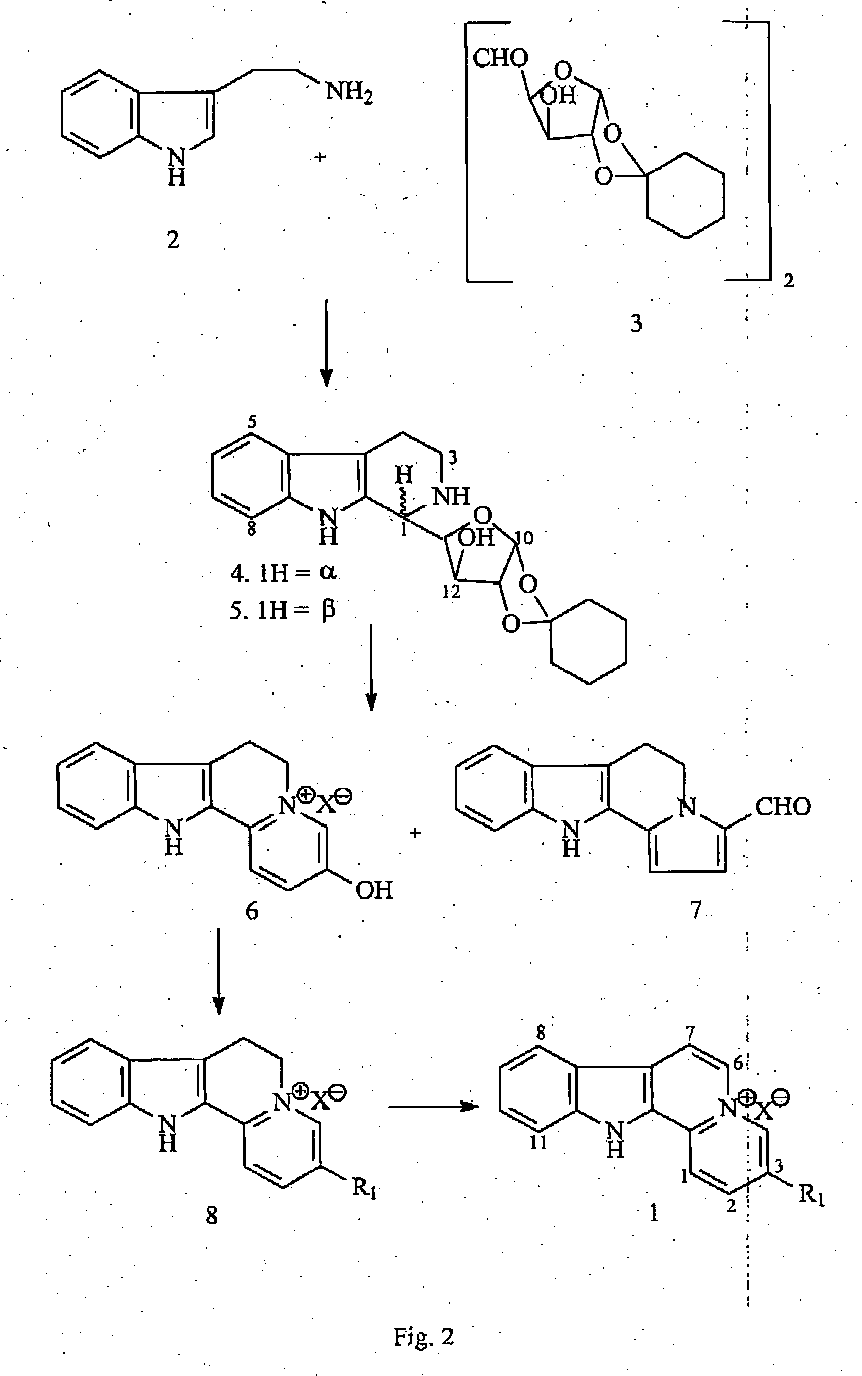

20. A method as claimed in claim 19 wherein said compound of formula 6 is oxidized to compounds of formula 9 in the presence of a dehydrogenating agent.

21. A process as claimed in claim 19 or 20 wherein inert solvent is selected from benzene, toluene, xylene.

22. A process as claimed in any one of the claims 19 to 21 wherein said acid is H₂SO₄, HOAc, or a mixture thereof.

23. A process as claimed in claim 20 wherein said dehydrogenating agent is Dichloro dicyano benzoquinone.

## Patentansprüche

1. Verbindung, ausgewählt unter (i) Verbindungen der Formel 1 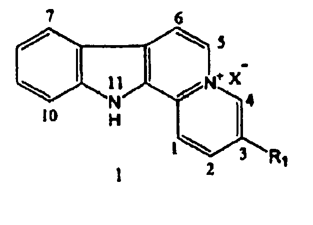 worin R₁ für Alkyl (nicht Ethyl), Alkoxy, Aryloxy, CH(OH)CH₃, CH(OAc)CH₃, CH(OCOC₆H₅)CH₃. CH(OBn)CH₃ und OBn steht, wobei Bn für CH₂C₆H₅ und X- für ein Gegenanion steht;
(ii) Verbindungen der Formel 8, worin R₁ für Alkyl (nicht Ethyl), Alkoxy, Aryloxy, CH(OAc)CH₃, CH(OCOC₆H₅)CH₃ und CH(OBn)CH₃ steht, worin Bn für CH₂C₆H₅ und X-für ein Gegenanion steht; 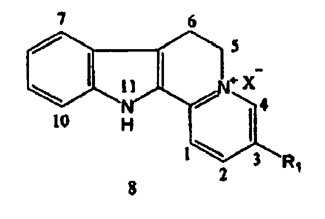
(iii) Verbindungen 22, 25, 26, 30 und 32, 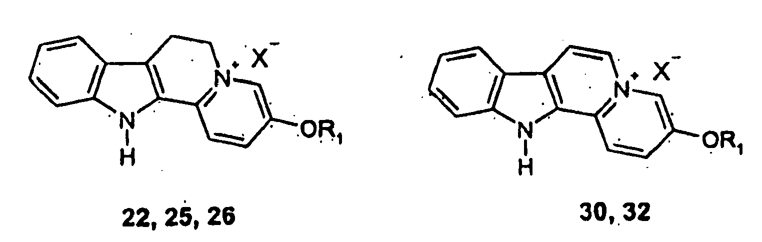
| Verbindung | R**₁** | X | Verbindung | R**₁** | X |
|---|---|---|---|---|---|
| **22** | H | HSO₄ | **30** | (CH2)₃Br | ClO₄ |
| **25** | CH₂C₆H₄F[4-] | ClO₄ | **32** | CH₂C₆H₄F[4-] | ClO₄ |
| **26** | CH₂C₆H₄Br[4-] | Br | | | |

2. Verbindung nach Anspruch 1 der Formel 13 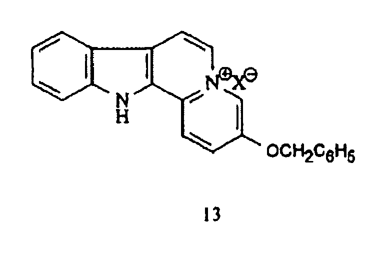 nämlich 3-Benzyloxyindolo[2,3-*a*]chinoliziniumsalz.

3. Verbindung nach Anspruch 1 der Formel 15 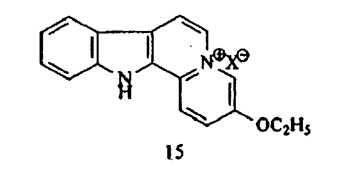 nämlich 3-Ethoxyindolo[2,3-*a*]chinoliziniumsalz.

4. Verbindung nach Anspruch 1 der Formel 19 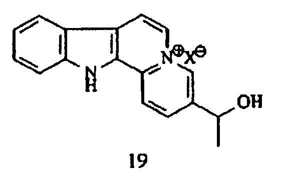 nämlich 3-(Hydroxyethyl)indolo[2,3-a]chinoliziniumsalz.

5. Verbindung nach Anspruch 1 der Formel 20 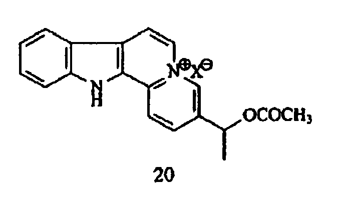 nämlich 3-(Acetoxyethyl)indolo[2,3-*a*]chinoliziniumsalz.

6. Verbindung nach Anspruch 1 der Formel 21 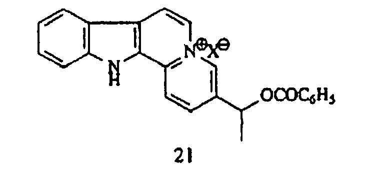 nämlich 3-(α-Benzoyloxyethyl)indolo[2,3-*a*]chinoliziniumsalz.

7. Verbindung nach Anspruch 1 der Formel 14 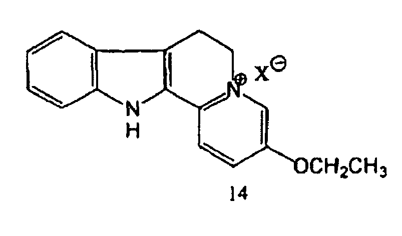 nämlich 3-Ethoxy-5,6-dihydroindolo[2,3-a]chinoliziniumsalz.

8. Verbindung nach Anspruch 1 der Formel 17 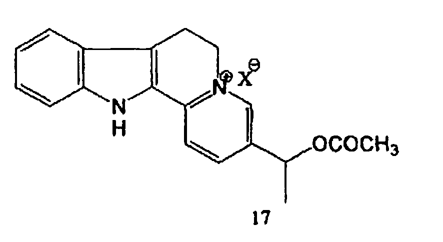 nämlich 3-(α-Acetoxyethyl)-5,6-dihydroindolo[2,3-a]chinoliziniumsalz.

9. Verbindung nach Anspruch 1 der Formel 18 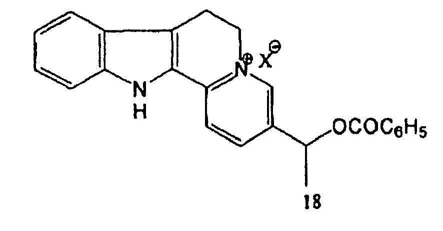 nämlich 3-(α-Benzoyloxyethyl)-5,6-dihydroindolo[2,3-*a*]chinoliziniumsalz.

10. Zusammensetzung, umfassend eine Verbindung nach einem der Ansprüche 1 bis 9 und einen pharmazeutisch verträglichen Hilfsstoff, Verdünnungsmittel, Exzipienten, Lösungsmittel, Binder, Stabilisator, Träger, Füllstoff oder Gleitmittel.

11. Zusammensetzung nach Anspruch 1, 2-6 und 10, die 0,1 bis 10 Gramm pro Einheitsdosis der Verbindung nach Formel 1 bereitstellt.

12. Zusammensetzung nach Anspruch 1, 7-9 und 10, die 0,1 bis 10 Gramm pro Einheitsdosis der Verbindung nach Formel 8 bereitstellt.

13. Zusammensetzung nach Anspruch 10-12 in Form einer Tablette, Pastille, Kapsel, Pulver, wässrigen oder öligen Suspension, Sirup, Elixier, Implantat oder wässrigen Lösung.

14. Verbindung nach einem der Ansprüche 1-9 zur Anwendung in einem Behandlungsverfahren eines Patienten mit Eierstock-, Brust-, Prostata-, Pankreas-, Mundhöhlen-, Lungen-, Darm- oder Kehlkopfkrebs, wobei man bei dem Verfahren eine wirksame Menge einer Verbindung nach einem der Ansprüche 1-9 oder einer Zusammensetzung nach einem der Ansprüche 10-13 dem bedürftigen Patienten verabreicht.

15. Verwendung einer Verbindung nach einem der Ansprüche 1-9 zur Herstellung eines Arzneimittels zur Anwendung in einem Behandlungsverfahren eines Patienten mit Eierstock-, Brust-, Prostata-, Pankreas-, Mundhöhlen-, Lungen-, Darm- oder Kehlkopfkrebs, wobei man bei dem Verfahren eine wirksame Menge einer Verbindung nach einem der Ansprüche 1-9 oder einer Zusammensetzung nach einem der Ansprüche 10-13 dem bedürftigen Patienten verabreicht.

16. Verwendung nach Anspruch 15, wobei der Patient ein Mensch, Säuger oder anderes Lebewesen ist.

17. Verwendung nach Anspruch 15 und 16, wobei die Dosierungshöhe für Menschen im Bereich von 1 mg/kg Körpergewicht bis 300 mg/kg Körpergewicht liegt.

18. Verwendung nach Ansprüchen 15-17, wobei man die Verbindung dem Patienten systemisch verabreicht.

19. Verfahren zur Herstellung von Indolo[2,3-a]chinoliziniumsalz der allgemeinen Formel 1 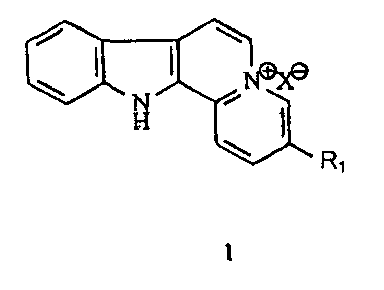 worin R₁ für Alkyl (nicht Ethyl), Alkoxy, Aryloxy, CH(OH)CH₃, CH(OAc)CH₃, CH(OBn)CH₃ steht, wobei Bn für CH₂C₆H₅ und X⁻ für ein Gegenanion steht, bei dem man ein Tryptamin der Formel 2 mit Di-(1,2-*O*-cyclohexyliden-α-D-xylopentadialdofuranose-5-hydrat)-5,5' : 3',5-dianhydrid der Formel 3 in einem inerten Lösungsmittel bei einer Temperatur im Bereich von 50 °C-150 °C über eine Dauer von 1 und 8 Stunden behandelt,
1-(10,11-O-Cyclohexyliden-12β-hydroxy-13β-tetrahydrofuranyl) tetrahydro-β-carbolin der Formel 4 und 1-(10,11-*O*-Cyclohexyliden-12β-hydroxy-13α-tetrahydrofuranyl) tetrahydro-β-carbolin der Formel 5 isoliert und reinigt,
die Verbindungen unter Bildung von Verbindungen der Formel 6 und 7 behandelt,
die Verbindung der Formel 6 zur Verbindung der Formel 8 derivatisiert
und die Verbindung der Formel 8 nach bekannten Verfahren in die Verbindung der Formel 1 umwandelt

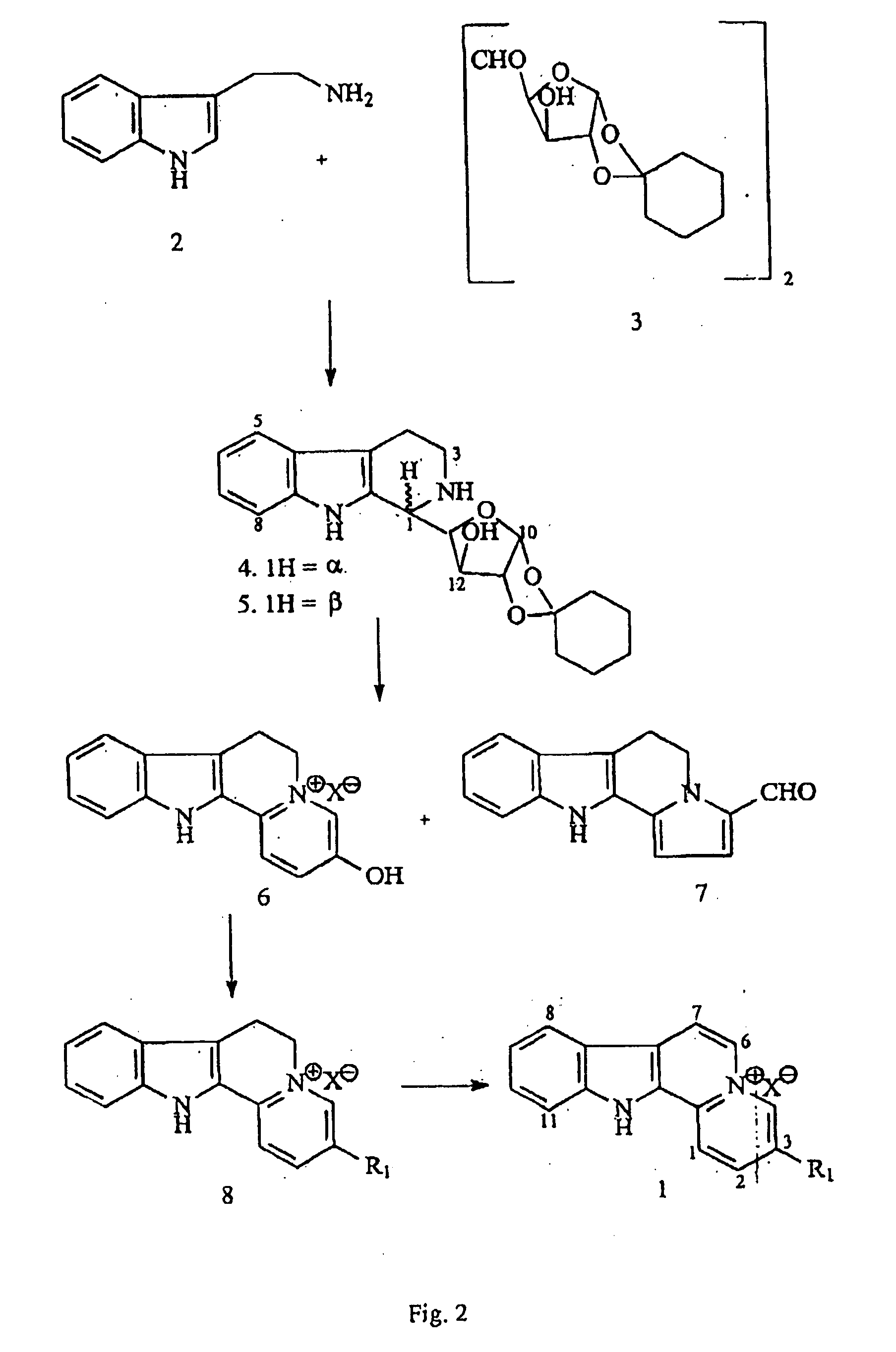

20. Verfahren nach Anspruch 19, wobei man die Verbindung der Formel 6 in Gegenwart eines Dehydriermittels zur Verbindung der Formel 9 oxydiert.

21. Verfahren nach Anspruch 19 oder 20, wobei das inerte Lösungsmittel unter Benzol, Toluol und Xylol ausgewählt ist.

22. Verfahren nach einem der Ansprüche 19 bis 21, wobei es sich bei der Säure um H₂SO₄, HOAc oder ein Gemisch davon handelt.

23. Verfahren nach Anspruch 20, wobei das Dehydriermittel Dichlordicyanobenzochinon ist.

## Revendications

1. Composé sélectionné parmi (i) les composés de la formule 1 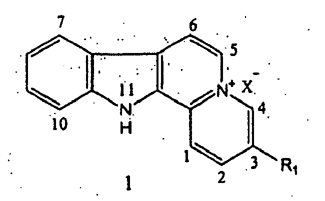 où R₁ représente alkyle (non éthyle), alkoxy, aryloxy, CH(OH)CH₃, CH(OAc)CH₃, CH(OCOC₆H₅)CH₃, CH(OBn)CH₃ et OBn, où Bn est CH₂C₆H₅ et X⁻ est un contre-anion ;
(ii) les composés de la formule 8, où R₁ représente alkyle (non éthyle), alkoxy, aryloxy, CH(OAc)CH₃, CH(OCOC₆H₅)CH₃, et CH(OBn)CH₃, où Bn est CH₂C₆H₅ et X⁻ est un contre-anion ; 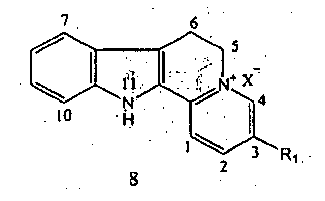
(iii) les composés 22, 25, 26, 30 et 32 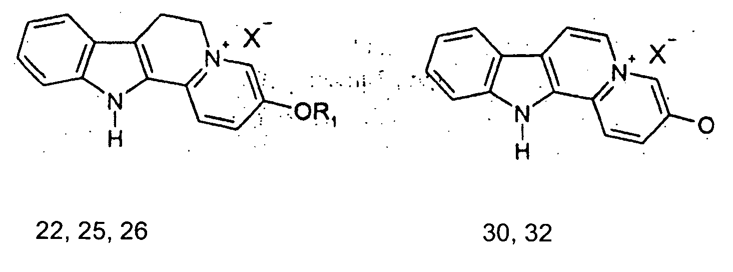
| Composé | R₁ | X | Composé | R₁ | X |
|---|---|---|---|---|---|
| 22 | H | HSO₄ | 30 | (CH₂)₃Br | ClO₄ |
| 25 | CH₂C₆H₄F[4-] | ClO₄ | 32 | CH₂C₆H₄F[4-] | ClO₄ |
| 26 | CH₂C₆H₄Br[4-] | Br | | | |

2. Composé selon la revendication 1, ayant la formule 13 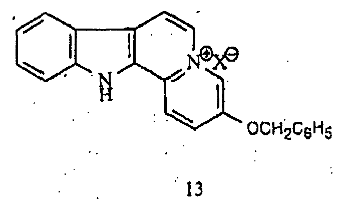 et qui est un sel de 3-benzyloxyindolo[2,3-*α*]quinolizinium.

3. Composé selon la revendication 1, ayant la formule 15 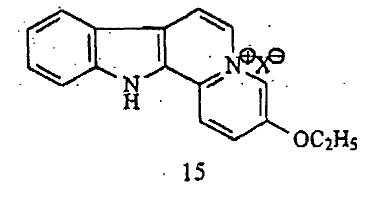 et qui est un sel de 3-éthoxyindolo[2,3-α]quinolizinium.

4. Composé selon la revendication 1, ayant la formule 19 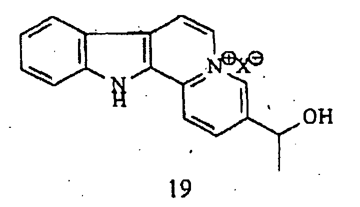 et qui est un sel de 3-(α-hydroxyéthyl)indolo[2,3-a]quinolizinium.

5. Composé selon la revendication 1, ayant la formule 20 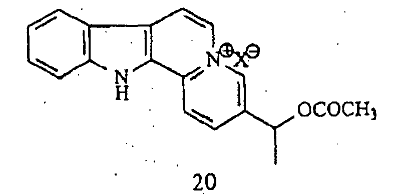 et qui est un sel de 3-(α-acétoxyéthyl)indolo[2,3-α]quinolizinium.

6. Composé selon la revendication 1, ayant la formule 21 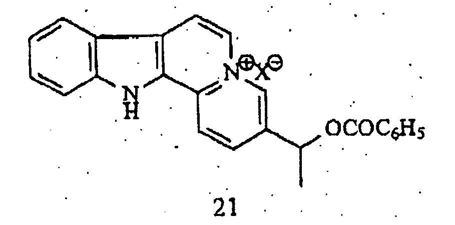 et qui est un sel de 3-(α-benzoyloxyéthyl)indolo[2,3-α]quinolizinium.

7. Composé selon la revendication 1, ayant la formule 14 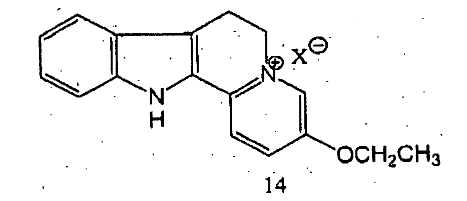 et qui est un sel de 3-éthoxy-5,6-dihydroindolo[2,3-α]quinolizinium.

8. Composé selon la revendication 1, ayant la formule 17 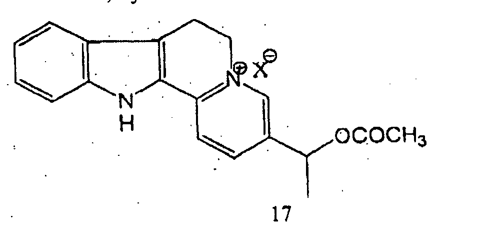 et qui est un sel de 3-(α-acétoxyéthyl)-5,6-dihydroindolo[2,3-α]quinolizinium.

9. Composé selon la revendication 1, ayant la formule 18 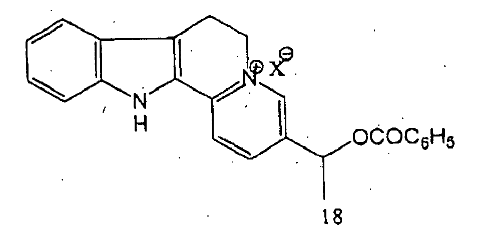 et qui est un sel de 3-(α-benzoyloxyéthyl)-5,6-dihydroindolo[2,3-α]quinolizinium.

10. Composition comprenant un composé selon l'une quelconque des revendications 1 à 9, et un additif, diluant, excipient, solvant, liant, stabilisant, entraîneur, charge ou lubrifiant pharmaceutiquement acceptable.

11. Composition selon les revendications 1, 2 à 6 et 10, qui fournit 0,1 à 10 grammes par unidose du composé de la formule 1.

12. Composition selon les revendications 1, 7 à 9 et 10, qui fournit 0,1 à 10 grammes par unidose du composé de la formule 8.

13. Composition selon les revendications 10 à 12, sous forme de comprimé, pastille, capsule, poudre, suspension aqueuse ou huileuse, sirop, élixir, implant ou solution aqueuse.

14. Composé selon l'une quelconque des revendications 1 à 9, destiné à être utilisé dans un procédé de traitement d'un patient atteint du cancer des ovaires, du sein, de la prostate, du pancréas, de la cavité orale, des poumons, du colon ou du larynx, ledit procédé comprenant l'administration d'une quantité efficace d'un composé selon l'une quelconque des revendications 1 à 9 ou d'une composition selon l'une quelconque des revendications 10 à 13 au patient qui en a besoin.

15. Utilisation d'un composé selon l'une quelconque des revendications 1 à 9 dans la fabrication d'un médicament destiné à être utilisé dans un procédé de traitement d'un patient atteint du cancer des ovaires, du sein, de la prostate, du pancréas, de la cavité orale, des poumons, du colon ou du larynx, ledit procédé comprenant l'administration d'une quantité efficace d'un composé selon l'une quelconque des revendications 1 à 9 ou d'une composition selon l'une quelconque des revendications 10 à 13 au patient qui en a besoin.

16. Utilisation selon la revendication 15, dans laquelle ledit patient est un humain, un mammifère ou autre animal.

17. Utilisation selon les revendications 15 et 16, dans laquelle le dosage pour les humains se situe dans la plage de 1 mg / Kg de poids corporel à 300 mg / Kg de poids corporel.

18. Utilisation selon les revendications 15 à 17, dans laquelle le composé est administré au patient de manière systémique.

19. Procédé pour la synthèse de sels d'indolo[2,3-α]quinolizinium de la formule générale 1 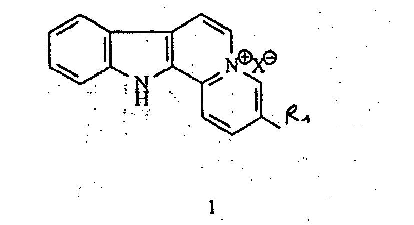 où R₁ représente alkyle (non éthyle), alkoxy, aryloxy, CH(OH)CH₃, CH(OAc)CH₃, CH(OBn)CH₃, où Bn est CH₂C₆Hₛ et X⁻ est un contre-anion, qui comprend les étapes consistant à :
traiter une tryptamine de la formule 2 avec du di-(1,2-*O*-cyclohexylidène-α-D-xylopentadialdofuranose-5-hydrate)-5,5' ; :3',5-dianhydride de la formule 3 dans un solvant inerte à une température située dans la plage de 50°C à 150°C pour une période située dans la plage de 1 à 8 heures,
isoler et purifier de la 1-(10,11-*O*-cyclohexylidène-12β-hydroxy-13β-tétrahydrofuranyl) tétrahydro-β-carboline de la formule 4 et de la 1-(10,11-*O*-cyclohexylidène-12β-hydroxy-13α-tétrahydrofuranyl)tétrahydro-β-carboline de la formule 5 ainsi produite,
traiter lesdits composés pour former des composés de la formule 6 et de la formule 7,
dérivatiser lesdits composés de la formule 6 en composés de la formule 8
et convertir lesdits composés de la formule 8 en composés de la formule 1 par des procédés connus :

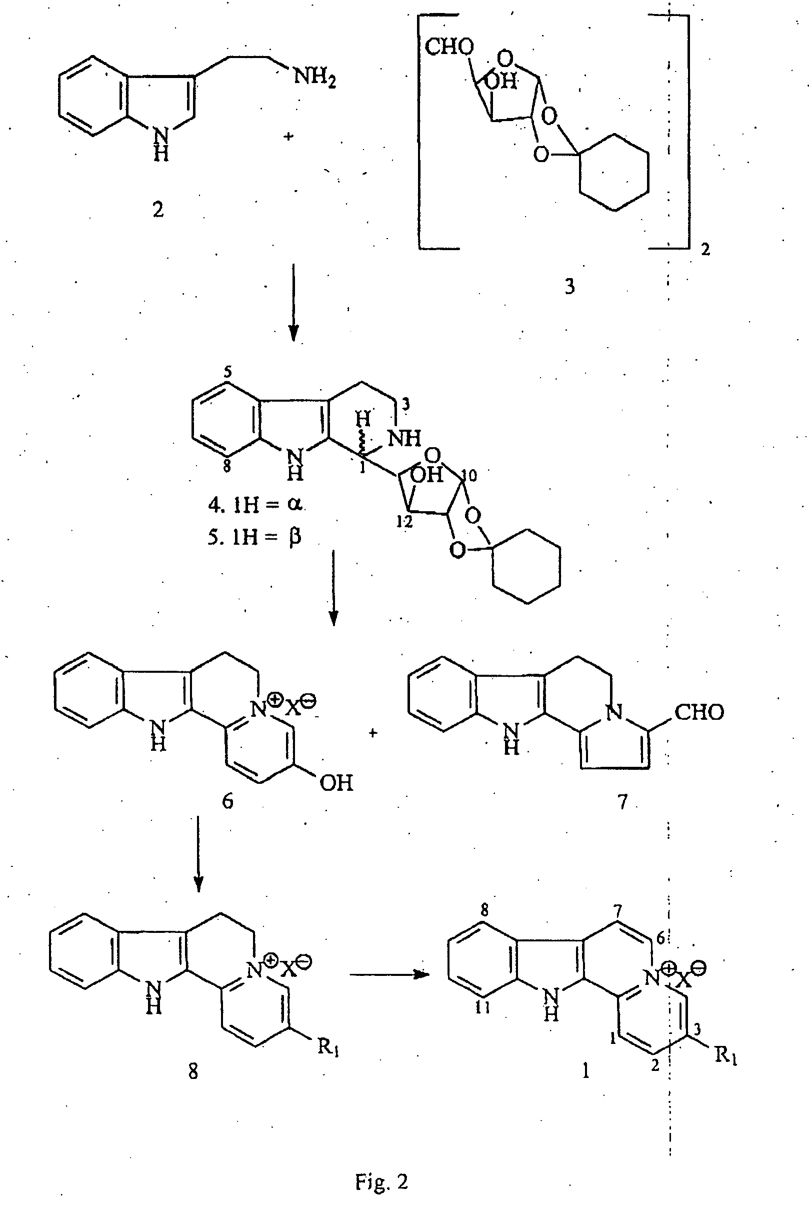

20. Procédé selon la revendication 19, dans lequel ledit composé de la formule 6 est oxydé en composés de la formule 9 en présence d'un produit de déshydrogénation,

21. Procédé selon la revendication 19 ou 20, dans lequel un solvant inerte est sélectionné parmi le benzène, le toluène ou le xylène.

22. Procédé selon l'une quelconque des revendications 19 à 21, dans lequel ledit acide est du H₂SO₄, du HOAc ou un mélange de ceux-ci.

23. Procédé selon la revendication 20, dans lequel ledit produit de déshydrogénation est de la dichloro dicyano benzoquinone.
